# EUROPEAN PATENT SPECIFICATION

(11) **EP 2 528 716 B1**
(45) Date of publication and mention of the grant of the patent: **06.05.2015**
(21) Application number: 11736671.6
(22) Date of filing: 28.01.2011
(51) Int. Cl.: B27B 1/00, G06K 9/00, B07C 5/14, G01N 33/46

(54) **METHOD FOR IDENTIFYING AN INDIVIDUAL LOG**
VERFAHREN ZUR IDENTIFIKATION EINES EINZELNEN BAUMSTAMMS
PROCÉDÉ D'IDENTIFICATION D'UNE GRUME INDIVIDUELLE

(30) Priority: 29.01.2010 FI 20105078
(43) Date of publication of application: 05.12.2012
(73) Proprietor: UPM-Kymmene Corporation, 00130 Helsinki (FI)
(72) Inventor: PÖLLÄNEN, Antti, FI-53300 Lappeenranta (FI); KANGASMAA, Tuomas, FI-28760 Pori (FI)
(74) Representative: Papula Oy
(86) International application number: PCT/FI2011/050071
(87) International publication number: WO 2011/092381

(56) References cited:
- WO-A1-2005/124323
- WO-A1-2005/124323
- FR-A1- 2 797 975
- US-A1- 2005 013 472
- US-A1- 2006 260 718
- US-A1- 2006 260 718
- US-B2- 7 426 422
- US-B2- 7 426 422

## Description

### FIELD OF THE INVENTION

The invention relates to the method defined in the preamble of claim 1 for identifying an individual log, wherein a property is measured from an individual log and the individual log is identified on the basis of the measured property.

### BACKGROUND OF THE INVENTION

Known from the prior art are different methods for sorting logs. Logs can be sorted in different categories on the basis of e.g. size, stem part, log quality and internal and external defects.

It is known from the prior art to sort the logs by visual machine vision methods. In connection with sorting the logs the classification is performed on the basis of external properties, e.g. dimensions, skew and ovality or color or their combinations.

Furthermore, known from the prior art is the identification of logs on the basis of an end image taken from one end of the log.

In addition, it is known from the prior art to identify an individual log by a RFID tag.

From US 2006/0260718, US 7426422 and WO 2005/124323 are known methods for identifying wood materials.

### OBJECTIVE OF THE INVENTION

An objective of the invention is to disclose a new type of a method for identifying and sorting an individual log. In addition, an objective of the invention is to disclose a method for utilizing at a later phase the properties measured earlier from an individual log, especially in connection with a sawing process.

### SUMMARY OF THE INVENTION

The method according to the invention is characterized by what has been presented in the claims.

The invention is based on a method for identifying an individual log, wherein a property is measured from an individual log and the individual log is identified on the basis of the measured property. According to the invention at least one predetermined property is measured from an individual log by a first identification device in order to determine at least one parameter substantially in connection with log sorting; the parameter determined from the individual log by the first identification device and preferably the measured properties are stored; at least one property is measured by the identification device, which property is selected from the group of: length of an individual log, diameter of an individual log, ovality of an individual log, tapering of an individual log and skew of an individual log and their combinations; a gravity centers curve is used as the parameter; the same predetermined parameter as determined by the first identification device is determined from the individual log in connection with sawing, in one preferred embodiment before sawing, by a second identification device; the parameter determined by the second identification device is compared with the stored parameter, on which basis the individual log is identified; and the properties stored from the individual log are collected after identification of the individual log and the collected data is utilized in planning the sawing of the individual log, especially in carrying out the sawing, and optimization of the sawing.

Planning the sawing signifies in this connection the optimal way of carrying out of the sawing of each individual log.

The invention is specifically based on identification of an individual log on the basis of internal and/or external properties. The individual log measured in connection with log sorting can be identified before sawing, in which case the properties and data measured in log sorting can be utilized in sawing of the log and optimization of the sawing of the log. In addition, the sawn timber formed by sawing can be identified. This way, the sawing process can be made substantially more effective by utilizing the measurement data of the earlier process steps, e.g. data of the internal properties of the log. In addition, the same properties need not be redetermined from an individual log in connection with the sawing and as the sawing process proceeds.

An individual log signifies in this connection any stem or a part thereof to be sawn via log sorting, such as the top log, middle log or butt log.

Log sorting signifies in this connection any sorting of a log either conventionally in entering the sawmill and at the sawmill or already in connection with cutting the log in the forest, in which case a suitable measuring instrument is provided in connection with the forestry equipment. In connection with log sorting, the desired properties are measured from an individual log and a parameter is determined.

In one embodiment more than one predetermined property is measured by the identification device from an individual log. Preferably the measured properties are stored e.g. in a database or equivalent from which the measured properties can be collected and utilized. In one embodiment the desired properties are measured by each identification device or some of the identification devices from an individual log.

In one embodiment of the invention more than one predetermined property is measured by the first identification device from an individual log. Preferably the measured properties are stored e.g. in the database or equivalent from which the measured properties can be collected and utilized.

In one embodiment of the invention more than one predetermined property is measured by the second identification device from an individual log. In one embodiment the measured properties are stored e.g. in the database or equivalent from which the measured properties can be collected and utilized.

In one embodiment at least one property is measured by the identification device, selected from the group of: length of an individual log, diameter of an individual log, ovality of an individual log, tapering of an individual log and skew of an individual log and their combinations.

In one embodiment of the invention more than one parameter is determined on which basis an individual log is identified in connection with the other identification device.

In one embodiment of the invention the parameter is selected from the group of dimensions of an individual log, different combinations of the dimensions, external properties of an individual log, different combinations of the external properties, end image and characteristics derived therefrom and/or gravity centers curve or their different combinations. Also other suitable parameters can be used. In one preferred embodiment the gravity centers curve is used as the parameter.

A gravity centers curve signifies in this connection a continuous curve formed via many gravity centers of densely measured cross-sectional surfaces. A gravity centers curve can also signify a continuous curve formed via the centers of the cross sections.

In one embodiment of the invention the second identification device is substantially similar to the first identification device. In an alternative embodiment the second identification device is a device which is different from the first identification device, but the same parameter can be determined with adequate reliability by the second identification device as by the first identification device.

In one embodiment at least one parameter is determined based on pattern recognition. Pattern recognition signifies identification and classification of objects application-specifically on the basis of defined properties based on external form. Pattern recognition can herein be carried out by machine vision, wherein the object is imaged by a camera and analyzed.

In one embodiment of the invention at least one parameter is determined by a log measuring instrument based on machine vision technology.

In one embodiment of the invention the same predetermined parameter as determined by the first identification device from an individual log is determined from sawn timber formed by sawing, and the determined parameter is compared with the stored parameter determined by the first device, on which basis the identification is made. In one embodiment the second identification device can be used as the identification device in order to determine the parameter from sawn timber formed by sawing. In an alternative embodiment a third identification device is used as the identification device in order to determine the parameter from sawn timber formed by sawing. In one embodiment the same predetermined parameter as determined from the individual log by the first or the second identification device is determined from sawn timber formed by sawing by the third identification device, and the parameter determined by the third device is compared with the previously determined and stored parameter, on which basis the identification is made. In one embodiment an end image is used as the parameter. In an alternative embodiment any parameter named in connection with this application can be used as the parameter.

In one embodiment the parameter can be determined from an individual log in connection with log sorting, such as cutting the individual log in the forest and/or in connection with entry at the sawmill, in connection with sawing and/or in connection with identification of sawn timber or in connection with their different combinations. In one embodiment the parameter can be determined from an individual log in connection with log sorting and in connection with sawing. In one embodiment the parameter can be determined from an individual log in connection with log sorting, in connection with sawing and in connection with the formed sawn timber. In one embodiment the parameter can be determined from an individual log in connection with log sorting in at least two different points, e.g. in connection with cutting the individual log in the forest and in connection with entry at the sawmill. In one embodiment the desired properties can be measured from an individual log in connection with any identification step and stored in the database.

In one embodiment the identification device is selected from the group of: devices based on camera technology, x-ray technology, laser technology, sound waves, microwaves, image analysis, devices based on texture analysis in order to identify individual features of wood and their different combinations. In one embodiment the identification device includes a line laser device and a CCD camera. The first identification device is preferably used in connection with log sorting and the second identification device is preferably used in identification of an individual log to be sawn before sawing, e.g. before peeling, after peeling, in connection with the log turner, in connection with feed of the sawing process and/or alternatively in identification of sawn timber.

In one embodiment the desired properties are measured from an individual log by at least one measuring instrument substantially in connection with identification of the individual log.

In one embodiment of the invention the desired properties are measured from an individual log by at least one measuring instrument substantially in connection with log sorting or one or more phases of log sorting.

In one embodiment of the invention the measuring instrument is a measuring instrument based on x-ray technology. In one embodiment the measuring instrument is a device based on x-ray technology, laser technology, sound waves, microwaves, digital imaging or image analysis or their combination.

In one embodiment at least one property is measured by the measuring instrument, selected from the group of: density, density distributions, gap between tree rings, part of bark, bark free diameter, bark free shape, sites of knots, gap between knots, sound/dead knot, part of heartwood, part of sapwood, quality of wood, durability of wood, amount of resin, decay, breaches and their combinations. Preferably the desired properties are measured by the measuring instrument from an individual log substantially in connection with log sorting.

In one embodiment of the invention the properties measured from an individual log in connection with identification, e.g. log sorting, are stored, e.g. in a data system or database. The measured properties can be utilized at a later phase, e.g. when an individual log has been identified for sawing.

In one embodiment the properties measured from an individual log by the first identification device and the parameter determined on that basis are stored, e.g. in the data system or database, and the parameter determined on the basis of the properties measured by the second identification device is compared with the earlier stored parameter, on which basis the individual log is identified.

In one embodiment the properties and measurement data stored from an individual log are collected, e.g. from the database, after identification of the individual log, and the collected data is utilized in planning and carrying out the sawing of the individual log. In one embodiment the sawing position of an individual log in entering the sawing is determined in the planning of the sawing, and the log rotation is performed on that basis. In one embodiment the control of log rotation is integrated with the control of planning of the sawing.

In addition the apparatus according to the invention includes a first identification device provided substantially in connection with log sorting in order to measure at least one predetermined property from an individual log in order to determine at least one parameter, and a second identification device located before the sawing process in order to determine the same predetermined parameter, and a data system in order to store, process and/or analyze the measured properties and the determined parameter and to identify the individual log on the basis of the parameter.

In one embodiment the data system includes computer software and a database in order to store, process and/or analyze the measured and/or determined data and to identify an individual log. Preferably the stored data is utilized in the sawing process and in the planning thereof and, in one embodiment, in identification of sawn timber. The employed database may be any database known per se and suitable for the purpose of use. Collection, storing, processing, analysis and reutilization of the measured properties can be controlled by any computer software known per se and suitable for the purpose of use.

Preferably the method according to the invention is used in connection with sorting and the sawing process of logs, preferably in the optimization of sawing. The invention can be used in the manufacture and identification of different sawn timber products.

Important advantages are achieved by the invention as compared with the prior art.

By the invention, an individual log can be identified easily and quickly and, at the same time, many properties can be determined from the individual log.

Thanks to the invention, optimal utilization of the raw material and each individual log in the sawing process is provided. In addition the data determined from an individual log follow the individual log from log sorting to the sawing process, and the properties need not be redetermined, thanks to the invention.

The invention provides for associating the raw material of a batch with a previously known raw material source, in which case traceability of the process can be ameliorated. Traceability in the entire chain offers new possibilities for quality surveillance, quality feedback and forest certification type needs and for process performance analysis.

By the invention, an industrially applicable easy, quick and useful manner of sorting an individual log and identifying it at a later phase is achieved.

### DETAILED DESCRIPTION OF THE INVENTION

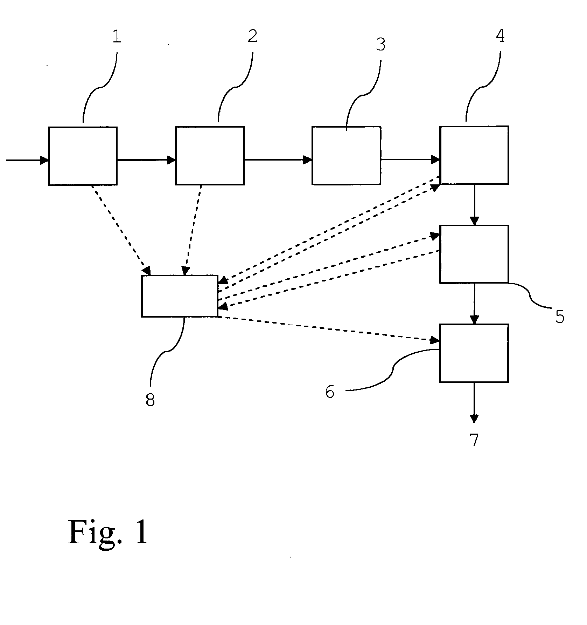

In the following, the invention will be described by detailed exemplary embodiments with reference to the accompanying figure 1.

### Example 1

Fig. 1 presents the process according to the invention for identifying an individual log.

In step 1 of the figure the predetermined properties are measured in connection with log sorting from an individual log by a first identification device which in this embodiment is a 3D device. The measured properties are stored in a data system 8. In the data system 8 a gravity centers curve is determined from the measured properties as a parameter which is also stored in the data system.

In step 2 of the figure other desired properties are measured from the individual log by a measuring instrument which in this embodiment is a device based on x-ray technology. The measured properties are stored in the data system 8.

In step 3 of the figure the individual log is stored temporarily.

In step 4 of the figure the same predetermined properties are measured from the individual log by a second identification device before sawing in order to determine the same parameter as determined by the first identification device in connection with log sorting. In this embodiment the employed second identification device is also a 3D device. The measured properties are stored in the data system 8 and used as a basis in order to determine a gravity centers curve that is also stored in the data system 8. The gravity centers curve determined by the second identification device is compared with the gravity centers curve stored in step 1, on which basis the individual log is identified.

This way the data and properties of the log measured in connection with log sorting in step 2 and stored in the data system 8 can be utilized in step 5 of the figure in sawing of the log and/or planning and optimization of sawing of the log. The individual log is provided in the correct sawing position by log rotation.

In step 6 of the figure the individual log is sawn according to step 5 in order to form sawn timber 7.

### Example 2

In this example the pieces of sawn timber, the individual log to which they belong, are identified by a camera system from the ends on the basis of an end image.

Previously it has not been possible to identify which pieces of sawn timber formed in sawing, such as center pieces and sideboards, are formed from which individual log in connection with a sawing process. Traceability can only be realized specifically for each sawing batch by the current methods. In one case the traceability and the pieces belonging to one specific log can at the most be determined by marking manually, which can only be carried out at a slower rate than the normal production.

In the method according to the invention the end of a log individual, i.e. a log, is imaged before sawing by a first identification device, and the sawing degrees and the rotation angle are added to the end image, or imaging of the end of the log is performed only after rotation, e.g. by a second identification device. This parameter is stored in a database. The equivalent end imaging is performed after sawing to the ends of the pieces of sawn timber, on which basis the identification is made. In connection with determining the parameters, other desired properties can be determined from the log at the same time and stored in the database. The end images of sawn products, their individual properties or parameters computed from them are compared with the equivalent data of the same point of time formed from the end images of the log and are used as a basis to determine which piece of sawn timber belongs to which log.

The method according to the invention lends itself as different embodiments to be used in most different identification, sorting and/or tracing applications in wood product industry.

The invention is not limited merely to the examples referred to above; instead, many variations are possible within the scope defined by the claims.

## Claims

1. A method for identifying an individual log, wherein a property is measured from an individual log and the individual log is identified on the basis of the measured property so that,
- at least one predetermined property is measured from an individual log by a first identification device in order to determine at least one parameter in connection with log sorting;
- the measured properties and the parameter determined from the individual log by the first identification device are stored;
- the same parameter as determined by the first identification device is determined from the individual log by a second identification device; and
- the parameter determined by the second identification device is compared with the stored parameter, on which basis the individual log is identified;
**characterized in that**
- at least one property is measured by the identification device, which property is selected from the group of: length of an individual log, diameter of an individual log, ovality of an individual log, tapering of an individual log and skew of an individual log and their combinations;
- a gravity centers curve is used as the parameter;
- the same parameter as determined by the first identification device is determined from the individual log by a second identification device before sawing and the individual log is identified, and
- the properties stored from the individual log are collected after identification of the individual log and the collected data is utilized in planning the sawing of the individual log and optimization of the sawing.

2. The method according to claim 1, **characterized in that** predetermined properties are measured by the first identification device from an individual log, and properties are selected from the group of: length of an individual log, diameter of an individual log, ovality of an individual log, tapering of an individual log and skew of an individual log and their combinations.

3. The method according to claim 1 or 2, **characterized in that** predetermined properties are measured by the second identification device from an individual log, and properties are selected from the group of: length of an individual log, diameter of an individual log, ovality of an individual log, tapering of an individual log and skew of an individual log and their combinations.

4. The method according to any one of claims 1 to 3, **characterized in that** more than one parameter is determined, on which basis an individual log is identified.

5. The method according to any one of claims 1 to 4, **characterized in that** the parameter is selected from the group of dimensions of an individual log, external properties of an individual log, end image, gravity centers curve and their combinations.

6. The method according to any one of claims 1 to 5, **characterized in that** at least one parameter is determined by a measuring instrument based on machine vision technology.

7. The method according to any one of claims 1 to 6, **characterized in that** the identification device is selected from the group of: devices based on camera technology, x-ray technology, laser technology, sound waves, microwaves, image analysis, devices based on texture analysis in order to identify individual features of wood and their different combinations.

8. The method according to any one of claims 1 to 7, **characterized in that** the second identification device is substantially similar to the first identification device.

9. The method according to any one of claims 1 to 8, **characterized in that** the desired properties are measured from an individual log by at least one measuring instrument substantially in connection with log sorting.

10. The method according to claim 9, **characterized in that** the properties measured from an individual log in connection with log sorting are stored.

11. The method according to claim 9 or 10, **characterized in that** the desired properties are measured from an individual log by a measuring instrument which is a device based on x-ray technology, laser technology, sound waves, microwaves, digital imaging or image analysis or their combination.

12. The method according to claim 9 or 10, **characterized in that** the desired properties are measured from an individual log by a measuring instrument based on x-ray technology.

13. The method according to any one of claims 1 to 12, **characterized in that** at least one property is measured by the measuring instrument, selected from the group of: density, density distributions, gap between tree rings, part of bark, bark free diameter, bark free shape, sites of knots, gap between knots, sound/dead knot, part of heartwood, part of sapwood, quality of wood, durability of wood, amount of resin, decay, breaches and their combinations.

14. The method according to any one of claims 1 to 13, **characterized in that** the same parameter as determined by the first identification device is determined from sawn timber formed by sawing, and the determined parameter is compared with the stored parameter, on which basis the identification is made.

## Patentansprüche

1. Verfahren zum Identifizieren eines einzelnen Baumstamms, wobei eine Eigenschaft eines einzelnen Baumstamms gemessen wird und der einzelne Baumstamm auf der Grundlage der gemessenen Eigenschaft identifiziert wird, so dass
- mindestens eine vorgegebene Eigenschaft eines einzelnen Baumstamms durch eine erste Identifikationsvorrichtung gemessen wird, um mindestens einen Parameter in Verbindung mit einer Baumstammsortierung zu bestimmen;
- die gemessenen Eigenschaften und der Parameter, der durch die erste Identifikationsvorrichtung von dem einzelnen Baumstamm bestimmt wird, gespeichert werden;
- derselbe Parameter, der durch die erste Identifikationsvorrichtung bestimmt wurde, durch eine zweite Identifikationsvorrichtung von dem einzelnen Baumstamm bestimmt wird und
- der von der zweiten Identifikationsvorrichtung bestimmte Parameter mit dem gespeicherten Parameter verglichen wird, wobei auf dieser Grundlage der einzelne Baumstamm identifiziert wird;
**dadurch gekennzeichnet, dass**
- durch die Identifikationsvorrichtung mindestens eine Eigenschaft gemessen wird, wobei diese Eigenschaft ausgewählt ist aus der Gruppe: Länge eines einzelnen Baumstamms, Durchmesser eines einzelnen Baumstamms, Ovalität eines einzelnen Baumstamms, Verjüngung eines einzelnen Baumstamms, Schräge eines einzelnen Baumstamms und deren Kombinationen;
- als der Parameter eine Schwerpunktkurve verwendet wird;
- derselbe Parameter, der durch die erste Identifikationsvorrichtung bestimmt wurde, vor dem Sägen durch eine zweite Identifikationsvorrichtung von dem einzelnen Baumstamm bestimmt wird und der einzelne Baumstamm identifiziert wird; und
- die zu dem einzelnen Baumstamm gespeicherten Eigenschaften nach der Identifikation des einzelnen Baumstamms gesammelt werden und die gesammelten Daten beim Planen des Sägens des einzelnen Baumstamms und bei der Optimierung des Sägens verwendet werden.

2. Verfahren nach Anspruch 1, **dadurch gekennzeichnet, dass** durch die erste Identifikationsvorrichtung vorgegebene Eigenschaften eines einzelnen Baumstamms gemessen werden und die Eigenschaften ausgewählt sind aus der Gruppe: Länge eines einzelnen Baumstamms, Durchmesser eines einzelnen Baumstamms, Ovalität eines einzelnen Baumstamms, Verjüngung eines einzelnen Baumstamms, Schräge eines einzelnen Baumstamms und deren Kombinationen.

3. Verfahren nach Anspruch 1 oder 2, **dadurch gekennzeichnet, dass** durch die zweite Identifikationsvorrichtung vorgegebene Eigenschaften eines einzelnen Baumstamms gemessen werden und die Eigenschaften ausgewählt sind aus der Gruppe: Länge eines einzelnen Baumstamms, Durchmesser eines einzelnen Baumstamms, Ovalität eines einzelnen Baumstamms, Verjüngung eines einzelnen Baumstamms, Schräge eines einzelnen Baumstamms und deren Kombinationen.

4. Verfahren nach einem der Ansprüche 1 bis 3, **dadurch gekennzeichnet, dass** mehr als ein Parameter bestimmt wird, auf deren Basis ein einzelner Baumstamm identifiziert wird.

5. Verfahren nach einem der Ansprüche 1 bis 4, **dadurch gekennzeichnet, dass** der Parameter aus der Gruppe aus Abmessungen eines einzelnen Baumstamms, äußeren Eigenschaften eines einzelnen Baumstamms, einer Endabbildung, einer Schwerpunktkurve und deren Kombinationen ausgewählt ist.

6. Verfahren nach einem der Ansprüche 1 bis 5, **dadurch gekennzeichnet, dass** mindestens ein Parameter durch ein Messinstrument auf der Grundlage einer maschinellen Bilderkennungstechnologie bestimmt wird.

7. Verfahren nach einem der Ansprüche 1 bis 6, **dadurch gekennzeichnet, dass** die Identifikationsvorrichtung ausgewählt ist aus der Gruppe aus: Vorrichtungen auf der Basis der Kameratechnologie, der Röntgentechnologie, der Lasertechnologie, von Schallwellen, von Mikrowellen, der Bildanalyse, Vorrichtungen auf der Basis der Texturanalyse, um einzelne Holzmerkmale zu identifizieren, und deren Kombinationen.

8. Verfahren nach einem der Ansprüche 1 bis 7, **dadurch gekennzeichnet, dass** die zweite Identifikationsvorrichtung der ersten Identifikationsvorrichtung im Wesentlichen ähnelt.

9. Verfahren nach einem der Ansprüche 1 bis 8, **dadurch gekennzeichnet, dass** die gewünschten Eigenschaften eines einzelnen Baumstamms durch mindestens ein Messinstrument im Wesentlichen in Verbindung mit einer Baumstammsortierung gemessen werden.

10. Verfahren nach Anspruch 9, **dadurch gekennzeichnet, dass** die Eigenschaften eines einzelnen Baumstamms, die in Verbindung mit einer Baumstammsortierung gemessen werden, gespeichert werden.

11. Verfahren nach Anspruch 9 oder 10, **dadurch gekennzeichnet, dass** die gewünschten Eigenschaften eines einzelnen Baumstamms durch ein Messinstrument gemessen werden, welches eine Vorrichtung auf der Basis der Röntgentechnologie, der Lasertechnologie, von Schallwellen, von Mikrowellen, der digitalen Abbildung oder der Bildanalyse oder deren Kombination ist.

12. Verfahren nach Anspruch 9 oder 10, **dadurch gekennzeichnet, dass** die gewünschten Eigenschaften eines einzelnen Baumstamms durch ein Messinstrument auf der Basis der Röntgentechnologie gemessen werden.

13. Verfahren nach einem der Ansprüche 1 bis 12, **dadurch gekennzeichnet, dass** durch das Messinstrument mindestens eine Eigenschaft gemessen wird, ausgewählt aus der Gruppe aus: Dichte, Dichteverteilungen, Lücke zwischen den Jahresringen, Rindenanteil, rindenfreier Durchmesser, rindenfreie Form, Positionen von Astknoten, Lücke zwischen Astknoten, gesunder/toter Astknoten, Kernholzanteil, Mantelholzanteil, Holzqualität, Haltbarkeit des Holzes, Harzmenge, Fäulnis, Risse und deren Kombinationen.

14. Verfahren nach einem der Ansprüche 1 bis 13, **dadurch gekennzeichnet, dass** derselbe Parameter, wie er durch die erste Identifikationsvorrichtung bestimmt wird, von gesägtem Holz bestimmt wird, das durch Sägen gebildet wird, und der bestimmte Parameter mit dem gespeicherten Parameter verglichen wird, auf dessen Basis die Identifikation vorgenommen wird.

## Revendications

1. Procédé d'identification d'une grume distincte, dans lequel une propriété est mesurée sur une grume distincte et la grume distincte est identifiée sur la base de la propriété mesurée de façon que :
- au moins une propriété prédéterminée est mesurée sur une grime distincte par un premier dispositif d'identification de façon à déterminer au moins un paramètre lié au triage de grumes,
- les propriétés mesurées et le paramètre déterminé sur la grume distincte par le premier dispositif d'identification sont mémorisés,
- le même paramètre que celui déterminé par le premier dispositif d'identification est déterminé sur la grume distincte par un second dispositif d'identification et
- le paramètre déterminé par le second dispositif d'identification est comparé au paramètre mémorisé, sur la base duquel la grume distincte est identifiée,
**caractérisé en ce que**
- au moins une propriété est mesurée par le dispositif d'identification, propriété, qui est sélectionnée parmi le groupe constitué par la longueur, le diamètre, l'ovalisation, la conicité et l'obliquité d'une grume distincte et leurs combinaisons,
- une courbe des centres de gravité est utilisée comme paramètre,
- le même paramètre que celui déterminé par le premier dispositif d'identification est déterminé sur la grume distincte par un second dispositif d'identification avant le sciage et la grume distincte est identifiée, et
- les propriétés mémorisées sur la grume distincte sont collectées après identification de la grume distincte et les données collectées sont utilisées pour le planning et l'optimisation du sciage de la grume distincte.

2. Procédé suivant la revendication 1, **caractérisé en ce que** des propriétés prédéterminées sont mesurées par le premier dispositif d'identification sur une grume distincte et des propriétés sont sélectionnées parmi le groupe constitué par la longueur, le diamètre, l'ovalisation, la conicité et l'obliquité d'une grume distincte et leurs combinaisons.

3. Procédé suivant la revendication 1 ou 2, **caractérisé en ce que** des propriétés prédéterminées sont mesurées par le second dispositif d'identification sur une grume distincte, et des propriétés sont sélectionnées parmi le groupe constitué par la longueur, le diamètre, l'ovalisation, la conicité et l'obliquité d'une grume distincte et leurs combinaisons.

4. Procédé suivant une quelconque des revendications 1 à 3, **caractérisé en ce que** sont déterminés plus d'un paramètre, sur la base desquels une grume distincte est identifiée.

5. Procédé suivant une quelconque des revendications 1 à 4, **caractérisé en ce que** le paramètre est sélectionné parmi le groupe constitué par les dimensions, les propriétés externes, l'aspect final, la courbe des centres de gravités d'une grume distincte et leurs combinaisons.

6. Procédé suivant une quelconque des revendications 1 à 5, **caractérisé en ce qu'**au moins un paramètre est déterminé par mesure à l'aide d'un instrument utilisant la technologie de vision industrielle.

7. Procédé suivant une quelconque des revendications 1 à 6, **caractérisé en ce que** le dispositif d'identification est sélectionné parmi le groupe constitué par des dispositifs utilisant la technologie de caméra, la technologie de radiographie, la technologie laser, les ondes de son, les micro-ondes, l'analyse d'images, les dispositifs utilisant l'analyse de textures pour identifier des caractéristiques distinctes du bois et leur différentes combinaisons.

8. Procédé suivant une quelconque des revendications 1 à 7, **caractérisé en ce que** le second dispositif d'identification est essentiellement similaire au premier dispositif d'identification.

9. Procédé suivant une quelconque des revendications 1 à 8, **caractérisé en ce que** les propriétés souhaitées sont mesurées sur une grume distincte par au moins un instrument de mesure essentiellement lié au triage des grumes.

10. Procédé suivant la revendication 9, **caractérisé en ce que** les propriétés liées au triage de grumes, mesurées sur la grume distincte, sont mémorisées.

11. Procédé suivant la revendication 9 ou 10, **caractérisé en ce que** les propriétés souhaitées sont mémorisées sur une grume distincte par un instrument de mesure qui est un dispositif utilisant la technologie de radiographie, la technologie laser, les ondes du son, les micro-ondes, l'imagerie numérique ou l'analyse d'images ou leurs combinaisons.

12. Procédé suivant la revendication 9 ou 10, **caractérisé en ce que** les propriétés souhaitées sont mesurées sur une grume distincte par un instrument de mesure utilisant la technologie de radiographie.

13. Procédé suivant une quelconque des revendications 1 à 12, **caractérisé en ce qu'**au moyen de l'instrument de mesure sélectionné est mesurée au moins une propriété parmi le groupe constitué par la densité, les distributions de densité, l'espace entre les cernes d'arbres, la partie d'écorce, le diamètre sans écorce, la forme sans écorce, les sites de noeuds, l'espace entre les sites de noeuds, les noeuds sains/morts, la partie de bois de coeur, la partie de l'aubier, la qualité du bois, la durabilité du bois, la teneur en résine, la dégradation, les fissures et leurs combinaisons.

14. Procédé suivant une quelconque des revendications 1 à 13, **caractérisé en ce que** le même paramètre que celui déterminé par le premier dispositif d'identification est déterminé sur du bois scié formé par sciage et le paramètre déterminé est comparé au paramètre mémorisé, sur la base duquel l'identification a lieu.
